# EUROPÄISCHE PATENTANMELDUNG

(11) **EP 4 850 592 A1**
(43) Veröffentlichungstag der Anmeldung: **09.12.2026**
(21) Anmeldenummer: 25181123.8
(22) Anmeldetag: 05.06.2025
(51) Int. Cl.: A61B 3/11, A61B 5/16

(54) **VERFAHREN ZUR ERMITTLUNG EINES KENNWERTS DER KOGNITIVEN BELASTUNG EINER PERSON**

(71) Anmelder: Soma Reality GmbH, 1020 Wien (AT)
(72) Erfinder: Gollan, Benedikt, 3420 Kritzendorf (AT)
(74) Vertreter: Hübscher & Partner Patentanwälte GmbH

(57) **Zusammenfassung**

Die Erfindung bezieht sich auf ein Verfahren zur Ermittlung eines Kennwerts (16) der kognitiven Belastung (13) einer Person (1) anhand pupillometrischer Messdaten, wobei in aufeinanderfolgenden Zeitschritten (15) je Zeitschritt (15) aus den Messdaten einer pupillometrischen Erfassungseinheit (3) ein die Pupillenweite der Person repräsentierender Pupillenwert (5), sowie aus den Messdaten eines Helligkeitssensors (4) ein die Helligkeit im Blickfeld (8) der Person (1) repräsentierender Helligkeitswert (7) abgeleitet wird, wobei die Helligkeitswerte (7) in Helligkeitsklassen (9) eingeteilt werden und die Pupillenwerte (5) der Helligkeitsklasse (9) des Helligkeitswerts (7) des jeweiligen Zeitschritts (15) zugeordnet werden. Um die Genauigkeit des Kennwerts (16) der kognitiven Belastung (13) zu verbessern wird vorgeschlagen, dass für jede Helligkeitsklasse (9) ein Referenzbereich (10) mit vorgegebener Breite so festgelegt wird, dass die größte Anzahl repräsentativer Pupillenwerte (5) der Helligkeitsklasse (9) innerhalb dieses Referenzbereichs (10) liegt, dass aus den Referenzbereichen (10) der Helligkeitsklassen (9) ein mit steigenden Helligkeitswerten (7) monotoner Referenzzusammenhang zwischen den Helligkeitswerten (7) und den die kleinstmöglichen Pupillenweiten repräsentierenden Pupillenwerten (12) ermittelt wird und dass für einen Zeitschritt (15) ein Referenzpupillenwert (12) über den Referenzzusammenhang aus dem Helligkeitswert (7) des Zeitschritts (15) bestimmt und der Kennwert (16) aus der Abweichung des Pupillenwerts (5) des Zeitschritts (15) vom Referenzpupillenwert (12) ermittelt wird.

## Beschreibung

Die Erfindung bezieht sich auf ein Verfahren zur Ermittlung eines Kennwerts der kognitiven Belastung einer Person anhand pupillometrischer Messdaten, wobei in aufeinanderfolgenden Zeitschritten je Zeitschritt aus den Messdaten einer pupillometrischen Erfassungseinheit ein die Pupillenweite der Person repräsentierender Pupillenwert, sowie aus den Messdaten eines Helligkeitssensors ein die Helligkeit im Blickfeld der Person repräsentierender Helligkeitswert abgeleitet wird, wobei die Helligkeitswerte in Helligkeitsklassen eingeteilt werden und die Pupillenwerte der Helligkeitsklasse des Helligkeitswerts des jeweiligen Zeitschritts zugeordnet werden.

Aus dem Stand der Technik sind Verfahren bekannt, bei denen die von der Helligkeit im Blickfeld der Person abhängigen Pupillenweiten gemessen werden, um über die Abweichung der Pupillenweite zu einer Referenzpupillenweite als Kennwert Rückschlüsse auf die kognitive Belastung der Person zu ziehen. Gerade bei sicherheitskritischen Aufgaben kann ein Kennwert der kognitiven Belastung durch eine objektive Risikobewertung und gegebenenfalls Ausgabe eines Warnsignals Unfälle vermeiden. Die zugrundeliegende Überlegung basiert auf der Erkenntnis, dass sich die Pupille bei kognitiver Belastung - etwa beim Lösen komplexer Aufgaben oder bei hoher Konzentration - messbar erweitert. Die gemessenen Pupillenweiten eines Zeitschritts werden Helligkeitsklassen gemessener Helligkeitswerte des Zeitschritts zugeordnet. Durch diese Helligkeitsklassen wird eine Sigmoidfunktion, die den Referenzzusammenhang zwischen den Helligkeitswerten und den Pupillenweiten im entspannten Zustand der Person abbilden sollte, erstellt. Anstelle der Pupillenweite selbst sind auch Verfahren beschrieben, bei denen die gemessene Pupillenweite in einen die Pupillenweite einer Person repräsentierenden Pupillenwert umgerechnet werden. Es hat sich herausgestellt, dass der Kennwert insbesondere bei niedrigen Helligkeiten zu einer fehlerhaften Einschätzung der kognitiven Belastung führt, die sich auch durch die Verwendung einer logarithmischen oder exponentiellen Funktion für die Ermittlung des Referenzzusammenhangs nicht zufriedenstellend verbessern lässt.

Der Erfindung liegt somit die Aufgabe zugrunde, bei einem Verfahren der eingangs beschriebenen Art die Genauigkeit des Kennwerts der kognitiven Belastung zu verbessern.

Die Erfindung löst die gestellte Aufgabe dadurch, dass für jede Helligkeitsklasse ein Referenzbereich mit vorgegebener Breite so festgelegt wird, dass die größte Anzahl repräsentativer Pupillenwerte der Helligkeitsklasse innerhalb dieses Referenzbereichs liegt, dass aus den Referenzbereichen der Helligkeitsklassen ein mit steigenden Helligkeitswerten monotoner Referenzzusammenhang zwischen den Helligkeitswerten und den die kleinstmöglichen Pupillenweiten repräsentierenden Pupillenwerten ermittelt wird und dass für einen Zeitschritt ein Referenzpupillenwert über den Referenzzusammenhang aus dem Helligkeitswert des Zeitschritts bestimmt und der Kennwert aus der Abweichung des Pupillenwerts des Zeitschritts vom Referenzpupillenwert ermittelt wird. Der Erfindung liegt die Erkenntnis zugrunde, dass zwar die beobachteten Pupillenweiten und die bestimmten Helligkeitswerte einem Messfehler unterliegen, der insbesondere bei einer relativen Helligkeitsmessung beträchtlich sein kann, dass aber die Pupillenweiten bei einer gleichbleibenden Helligkeit, abgesehen von physiologischen Anpassungseffekten, durch kognitive Belastung nur in einem vorgegebenen Referenzbereich variieren können, der von physiologischen Gegebenheiten abhängt und individuell in geringem Ausmaß abweicht. Dies bedeutet, dass Pupillenweiten, die außerhalb eines solchen Referenzbereiches liegen, auf einen Messfehler oder physiologische Anpassungseffekte zurückzuführen sind. Wird nun der Referenzbereich innerhalb jeder Helligkeitsklasse so festgelegt, dass die größte Anzahl repräsentativer Pupillenwerte der Helligkeitsklasse innerhalb dieses Referenzbereichs liegt, können die Grenzen des Referenzbereichs die durch kognitive Belastung induzierte Pupillenweitenänderung festlegen. Als repräsentative Pupillenwerte können die bestimmten Qualitätskriterien genügenden Pupillenwerte, die der jeweiligen Helligkeitsklasse zugeordnet wurden, ausgewählt werden. Die untere Grenze des Referenzbereichs wird für die Ermittlung des Referenzzusammenhangs als der die kleinstmögliche Pupillenweite repräsentierende Pupillenwert bei geringster bislang beobachteter kognitiver Belastung angenommen. Neben diesen auf eine bestimmte Helligkeit bezogenen Aspekten kann die Genauigkeit des Kennwerts weiter erhöht werden, indem die beobachteten, die kleinstmöglichen Pupillenweiten repräsentierenden Pupillenwerte benachbarter Helligkeitsklassen mitberücksichtigt werden. Dies erfolgt erfindungsgemäß durch die Bedingung, dass die die kleinstmöglichen Pupillenweiten repräsentierenden Pupillenwerte benachbarter Helligkeitsklassen mit der Helligkeit monoton verlaufen müssen. Für den Fall, dass sich der Pupillenwert zur Pupillenweite direkt proportional verhält, wäre dieser Verlauf beispielsweise monoton fallend. Repräsentiert der Pupillenwert unmittelbar eine sich aus der Pupillenweite ergebende, absolute Helligkeit, so wäre dieser Verlauf monoton steigend. Zusammengefasst werden durch die Festlegung der Referenzbereiche und die Bedingung eines monotonen Referenzzusammenhangs zwischen den die kleinstmöglichen Pupillenweiten repräsentierenden Pupillenwerten und der Helligkeit Pupillenwerte, die nicht im Rahmen der physiologischen Plausibilität für die jeweilige Helligkeitsklasse selbst und benachbarter Helligkeitsklassen sind aus der Ermittlung des Referenzzusammenhangs ausgeschlossen, was zu einer Bereinigung der Datensätze und somit einer erhöhten Genauigkeit führt. Der Referenzzusammenhang zwischen den Helligkeitswerten und den die kleinstmöglichen Pupillenweiten repräsentierenden Pupillenwerte kann als Lookup-Tabelle abgespeichert und somit für jeden Helligkeitswert ein zugeordneter Referenzpupillenwert abgerufen werden. Für den abgeleiteten Helligkeitswert eines Zeitschritts kann somit über die Abweichung des Pupillenwerts des Zeitschritts vom abgerufenen Referenzpupillenwert ein Kennwert der kognitiven Belastung einer Person zu diesem Zeitschritt ermittelt und bevorzugt ausgegeben werden. Liegt der Kennwert außerhalb eines vorgegebenen Kennwertbereichs, so kann ein Warnsignal ausgegeben werden. Der Helligkeitswert kann eine von der Kamera relativ gemessene Helligkeit sein, oder in Kenntnis der Kameraparameter wie insbesondere der Blendenzahl auch eine absolute Helligkeit sein.

Versuche haben gezeigt, dass sich besonders genaue Kennwerte ermitteln lassen, wenn die vorgegebene Breite des Referenzbereichs in einem Bereich von 0,70 - 1,00 mm, bevorzugt 0,70 - 0,90 mm, insbesondere bevorzugt 0,75 - 0,85 mm liegt.

Um die Genauigkeit des Kennwerts insbesondere bei geringen Helligkeitswerten zu verbessern, kann der monotone Referenzzusammenhang durch stückweise lineare Interpolationen ermittelt werden. Im Rahmen der Erfindung wurde nämlich festgestellt, dass die Pupille bei einer geringen Helligkeit maximal erweitert ist und bereits minimale Lichtreize zu einer überproportionalen Änderung der Pupillenweite führen können. Dadurch führen die aus dem Stand der Technik bekannten Interpolationsverfahren zur Beschreibung des Referenzzusammenhangs zu keiner oder insbesondere bei geringen Helligkeitswerten zu ungenauen Lösungen. Durch die stückweise lineare Interpolation wird dem Referenzzusammenhang keine zugrundeliegende Funktionsform, und somit keine fehleranfällige Datenextrapolation aufgeprägt. Dies ist insbesondere dann wichtig, wenn nicht alle Helligkeitsklassen Pupillenwerte aufweisen, die statistisch signifikant sind oder wenn vorhandene Pupillenwerte den monotonen Referenzzusammenhang nicht erlauben würden.

Um den Referenzzusammenhang rascher an veränderliche Umgebungsbedingungen anpassen und Störungen zuverlässiger entfernen zu können, wird vorgeschlagen, dass die repräsentativen Pupillenwerte durch Gewichtung der der jeweiligen Helligkeitsklasse zugeordneten Pupillenwerte ermittelt werden.

Um beispielsweise den Einfluss störender Einmaleffekte zu reduzieren, können die der jeweiligen Helligkeitsklasse zugeordneten Pupillenwerte zur Ermittlung der repräsentativen Pupillenwerte nach ihrer statistischen Signifikanz gewichtet werden. In einer bevorzugten Ausführungsform können als repräsentative Pupillenwerte jene Pupillenwerte einer Helligkeitsklasse herangezogen werden, die außerhalb der größten und kleinsten 10%, insbesondere außerhalb der größten und kleinsten 5% aller repräsentierten Pupillenweiten der Helligkeitsklasse liegen. Auf diese Weise können kurzzeitige Helligkeitsänderungen, wie beispielsweise Lichtreflexe oder statistisch insignifikante physiologische Reaktionen in einfacher Weise unberücksichtigt bleiben, ohne die exakte Verteilung der Pupillenwerte innerhalb der jeweiligen Helligkeitsklasse ressourcenintensiv abschätzen zu müssen.

Um den Referenzzusammenhang möglichst exakt für die zu den jeweiligen Zeitschritten vorherrschende Helligkeit ermitteln zu können, können die Pupillenwerte den Helligkeitsklassen gemeinsam mit einer Zeitschrittinformation zugeordnet werden und die der jeweiligen Helligkeitsklasse zugeordneten Pupillenwerte zur Ermittlung der repräsentativen Pupillenwerte nach der Anzahl der vergangenen Zeitschritte gewichtet werden. Dadurch kann eine raschere Adaption an veränderliche Umgebungsbedingungen erfolgen, sodass für die aktuellen vorherrschenden Umgebungsbedingungen vergleichsweise mehr Messwerte zur Verfügung stehen, die den Referenzzusammenhang stärker stützen. Insbesondere kann dies bedeuten, dass nach einer bestimmten Anzahl vergangener Zeitschritte Pupillenwerte so gewichtet werden, dass sie gar nicht mehr zur Ermittlung der repräsentativen Pupillenwerte herangezogen werden.

Um insbesondere im Fall einer stärkeren Gewichtung der kürzlich abgeleiteten Pupillenwerte den Einfluss störender Einmaleffekte zu reduzieren, können die abgeleiteten Pupillenwerte nur dann der jeweiligen Helligkeitsklasse zugeordnet werden, wenn eine vorgegebene Anzahl zuvor gemessener Helligkeitswerte innerhalb einer vorgegebenen Helligkeitsschwankungsbreite liegt. Diese Maßnahme geht von der Überlegung aus, dass sich störende Einmaleffekte in kurzfristigen, signifikanten Änderungen der gemessenen Helligkeitswerte niederschlagen und gleichbleibende Helligkeitswerte auf stabile Umgebungsbedingungen hindeuten, auf die bereits eine physiologische Adaption erfolgt ist. Somit können Überreaktionen der Pupillenweite, wie sie beim Übergang zwischen verschiedenen Helligkeiten beobachtet wurden, herausgefiltert werden.

Da sich herausgestellt hat, dass die Pupillenreaktion aufgrund ihrer Nichtlinearität über verschiedene Helligkeiten nicht den im Stand der Technik vorgeschlagenen Interpolationsverfahren folgt, können die Referenzbereiche entsprechend der Anzahl und/oder Verteilung der Pupillenwerte im jeweiligen Referenzbereich gewichtet werden und zur Ermittlung des Referenzzusammenhangs diejenigen Referenzbereiche herangezogen werden, die insgesamt die höchste Gewichtung aufweisen. Dadurch werden die Referenzbereiche, für die die meisten tatsächlich gemessenen Pupillenwerte vorliegen, bevorzugt zur Ermittlung des Referenzzusammenhangs herangezogen.

Um eine Aussage darüber treffen zu können, wie verlässlich der Kennwert ist, kann für jeden Helligkeitswert des Referenzzusammenhangs ein Validitätswert bestimmt werden, der angibt, wie sehr der Referenzzusammenhang bei diesem Helligkeitswert von den bislang ermittelten Pupillenwerten gestützt ist und gemeinsam mit dem über den Referenzpupillenwert bei diesem Helligkeitswert ermittelten Kennwert ausgegeben werden.

Die Erfindung bezieht sich auch auf eine tragbare Vorrichtung zur Durchführung des beschriebenen Verfahrens umfassend eine pupillometrische Erfassungseinheit zur Ableitung eines Pupillenwerts und einen Helligkeitssensor zur Ableitung eines die Helligkeit im Blickfeld der Person repräsentierenden Helligkeitswerts. Insbesondere können die pupillometrische Erfassungseinheit und der Helligkeitssensor an einer von einer Person tragbaren Brille angeordnet werden.

In der Zeichnung ist der Erfindungsgegenstand beispielsweise dargestellt. Es zeigen
Fig. 1 eine schematische Anordnung einer Vorrichtung zur Durchführung des erfindungsgemäßen Verfahrens,
Fig. 2 ein Diagramm welches die abgeleiteten Pupillenwerte und Helligkeitswerte eines jeweiligen Zeitschritts abbildet und
Fig. 3 ein Diagramm, welches den Helligkeitswert, den Pupillenwert, den Referenzpupillenwert, den Kennwert und eine kognitive Belastung im zeitlichen Verlauf abbildet.

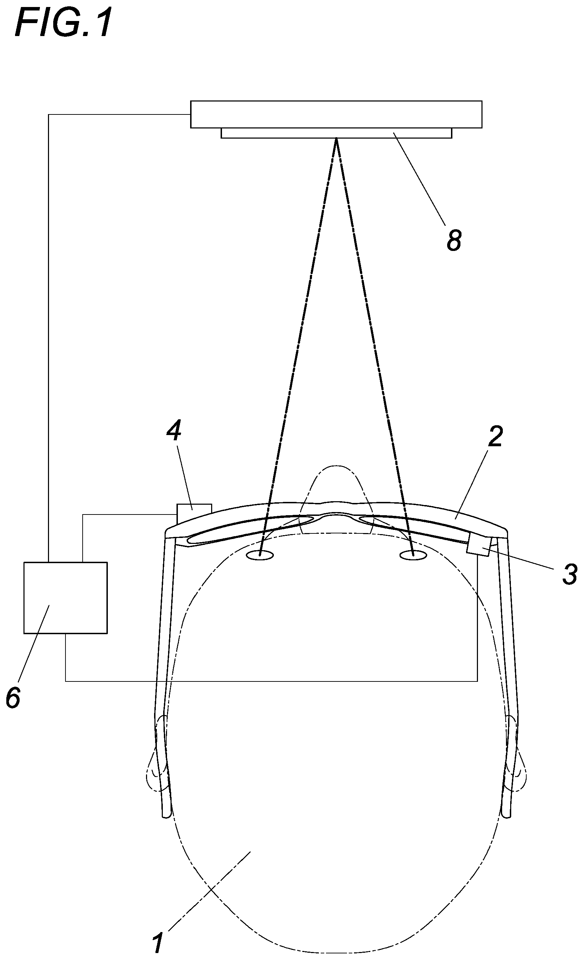

Fig. 1 zeigt eine Person 1, welche eine Brille 2 umfassend eine pupillometrische Erfassungseinheit 3, sowie einen Helligkeitssensor 4 aufweist. Aus den Messdaten der pupillometrischen Erfassungseinheit 3 wird je Zeitschritt ein die Pupillenweite der Person 1 repräsentierender Pupillenwert 5 abgeleitet und an eine Recheneinheit 6 weitergegeben. Aus den Messdaten des Helligkeitssensors 4 wird je Zeitschritt ein Helligkeitswert 7 im Blickfeld 8 der Person 1 abgeleitet und an die Recheneinheit 6 weitergegeben.

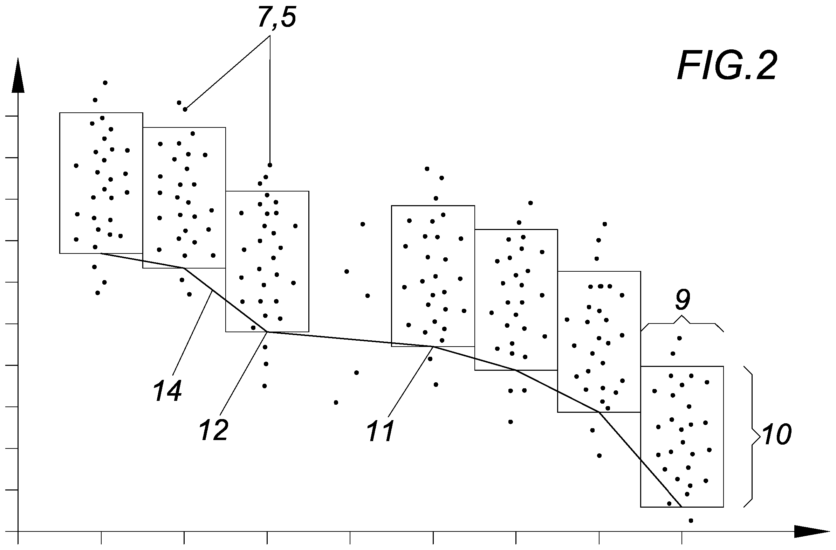

Fig. 2 zeigt ein Diagramm, in welchem die gemessenen Helligkeitswerte 7 und Pupillenwerte 5 eines jeden Zeitschritts abgebildet sind. Die Helligkeitswerte 7 werden in Helligkeitsklassen 9 eingeteilt. Die Pupillenwerte 5 des Diagramms verhalten sich mit der Pupillenweite direkt proportional. Für jede Helligkeitsklasse 9 wird ein Referenzbereich 10 mit einer vorgegebenen Breite so festgelegt, dass die größte Anzahl repräsentativer Pupillenwerte 5 der Helligkeitsklasse 9 innerhalb des Referenzbereichs 10 liegt. In der Figur werden die untersten Grenzen 11 der Referenzbereiche 10 als der die kleinstmögliche Pupillenweite repräsentierende Pupillenwert (Referenzpupillenwert) 12 bei geringster bislang beobachteter kognitiver Belastung 13 für die jeweilige Helligkeitsklasse 9 angenommen und durch stückweise lineare Interpolation 14 zwischen den untersten Grenzen 11 der Referenzbereiche 10 ein in diesem Fall monoton fallender Referenzzusammenhang zwischen den Helligkeitswerten 7 und den die kleinstmögliche Pupillenweite repräsentierenden Pupillenwerten 12 ermittelt. Zur Ermittlung des Referenzzusammenhangs werden nur jene Referenzbereiche 10 herangezogen, für die es eine ausreichende Anzahl an Pupillenwerten 5 gibt und die die Monotonie des Referenzzusammenhangs nicht stören.

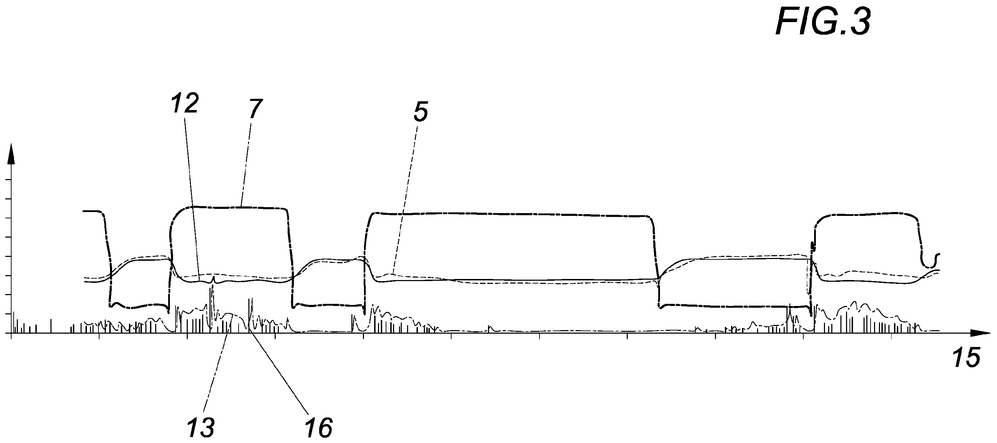

Figur 3 zeigt im zeitlichen Verlauf für jeden Zeitschritt 15 den abgeleiteten Helligkeitswert 7, den abgeleiteten, die Pupillenweite repräsentierend Pupillenwert 5, den aus dem Referenzzusammenhang aus dem Helligkeitswert des Zeitschritts bestimmten Referenzpupillenwert 12, den aus der Abweichung des Pupillenwerts 5 vom Referenzpupillenwert 12 ermittelten Kennwert 16 und eine über den Kennwert ermittelte kognitive Belastung 13. In diesem Fall verhält sich der Pupillenwert 5 direkt proportional zur Pupillenweite, weshalb bei einem steigenden Helligkeitswert 7 der Pupillenwert 5 kleiner wird. Über die Abweichung zwischen dem Referenzpupillenwert 12, der die kleinstmögliche Pupillenweite für den Helligkeitswert des Zeitschritts angibt, und dem für diesen Zeitschritt 15 abgeleiteten Pupillenwert 5 lässt sich ein Kennwert 16 für die kognitive Belastung 13 der Person zu diesem Zeitschritt 15 ermitteln, da sich die Pupillenweite je nach kognitiver Belastung 13 vergrößert.

## Patentansprüche

1. Verfahren zur Ermittlung eines Kennwerts 16 der kognitiven Belastung 13 einer Person 1 anhand pupillometrischer Messdaten, wobei in aufeinanderfolgenden Zeitschritten 15 je Zeitschritt 15 aus den Messdaten einer pupillometrischen Erfassungseinheit 3 ein die Pupillenweite der Person repräsentierender Pupillenwert 5, sowie aus den Messdaten eines Helligkeitssensors 4 ein die Helligkeit im Blickfeld 8 der Person 1 repräsentierender Helligkeitswert 7 abgeleitet wird, wobei die Helligkeitswerte 7 in Helligkeitsklassen 9 eingeteilt werden und die Pupillenwerte 5 der Helligkeitsklasse 9 des Helligkeitswerts 7 des jeweiligen Zeitschritts 15 zugeordnet werden, **dadurch gekennzeichnet, dass** für jede Helligkeitsklasse 9 ein Referenzbereich 10 mit vorgegebener Breite so festgelegt wird, dass die größte Anzahl repräsentativer Pupillenwerte 5 der Helligkeitsklasse 9 innerhalb dieses Referenzbereichs 10 liegt, dass aus den Referenzbereichen 10 der Helligkeitsklassen 9 ein mit steigenden Helligkeitswerten 7 monotoner Referenzzusammenhang zwischen den Helligkeitswerten 7 und den die kleinstmöglichen Pupillenweiten repräsentierenden Pupillenwerten 12 ermittelt wird und dass für einen Zeitschritt 15 ein Referenzpupillenwert 12 über den Referenzzusammenhang aus dem Helligkeitswert 7 des Zeitschritts 15 bestimmt und der Kennwert 16 aus der Abweichung des Pupillenwerts 5 des Zeitschritts 15 vom Referenzpupillenwert 12 ermittelt wird.

2. Verfahren nach Anspruch 1, **dadurch gekennzeichnet, dass** die vorgegebene Breite des Referenzbereichs 10 in einem Bereich von 0,70 - 1,00 mm liegt.

3. Verfahren nach Anspruch 1 oder 2, **dadurch gekennzeichnet, dass** der monotone Referenzzusammenhang durch stückweise lineare Interpolationen 14 ermittelt wird.

4. Verfahren nach einem der Ansprüche 1 bis 3, **dadurch gekennzeichnet, dass** die repräsentativen Pupillenwerte 5 durch Gewichtung der der jeweiligen Helligkeitsklasse 9 zugeordneten Pupillenwerte 5 ermittelt werden.

5. Verfahren nach Anspruch 4, **dadurch gekennzeichnet, dass** die der jeweiligen Helligkeitsklasse 9 zugeordneten Pupillenwerte 5 zur Ermittlung der repräsentativen Pupillenwerte 5 nach ihrer statistischen Signifikanz gewichtet werden.

6. Verfahren nach Anspruch 4 oder 5, **dadurch gekennzeichnet, dass** die Pupillenwerte 5 den Helligkeitsklassen 9 gemeinsam mit einer Zeitschrittinformation zugeordnet werden und die der jeweiligen Helligkeitsklasse 9 zugeordneten Pupillenwerte 5 zur Ermittlung der repräsentativen Pupillenwerte 5 nach der Anzahl der vergangenen Zeitschritte 15 gewichtet werden.

7. Verfahren nach einem der Ansprüche 1 bis 6, **dadurch gekennzeichnet, dass** die abgeleiteten Pupillenwerte 5 nur dann der jeweiligen Helligkeitsklasse 9 zugeordnet werden, wenn eine vorgegebene Anzahl zuvor gemessener Helligkeitswerte 7 innerhalb einer vorgegebenen Helligkeitsschwankungsbreite liegt.

8. Verfahren nach einem der Ansprüche 1 bis 7, **dadurch gekennzeichnet, dass** die Referenzbereiche 10 entsprechend der Anzahl und/oder Verteilung der Pupillenwerte 5 im jeweiligen Referenzbereich 10 gewichtet werden und zur Ermittlung des Referenzzusammenhangs diejenigen Referenzbereiche 10 herangezogen werden, die insgesamt die höchste Gewichtung aufweisen.

9. Verfahren nach Anspruch 8, **dadurch gekennzeichnet, dass** für jeden Helligkeitswert 7 des Referenzzusammenhangs ein Validitätswert bestimmt wird, der angibt, wie sehr der Referenzzusammenhang bei diesem Helligkeitswert 7 von den bislang ermittelten Pupillenwerten 5 gestützt ist und gemeinsam mit dem über den Referenzpupillenwert 12 bei diesem Helligkeitswert 7 ermittelten Kennwert 16 ausgegeben wird.
